# EUROPEAN PATENT APPLICATION

(11) **EP 2 628 480 A1**
(43) Date of publication of application: **21.08.2013**
(21) Application number: 12162306.0
(22) Date of filing: 29.03.2012
(51) Int. Cl.: A61K 31/122, A61K 36/07, A61P 35/00

(54) **Compounds and pharmaceutical compositions thereof for inhibiting mammalian tumor cell proliferation**

(30) Priority: 20.02.2012 TW 101105469
(71) Applicant: China Medical University, Taichung City 404 (TW)
(72) Inventor: Hseu, You-Cheng, Taichung City (TW); Yang, Hsin-Ling, Taichung City (TW)
(74) Representative: Lang, Christian

(57) **Abstract**

Compounds of following general formula (I) and pharmaceutical compositions thereof for inhibiting mammalian tumor cell proliferation are disclosed. In formula (I), n is an integer from 0 to 9. The cell proliferation of breast tumor, ovarian tumor, skin tumor or liver tumor is inhibited by the compounds of the formula (I).

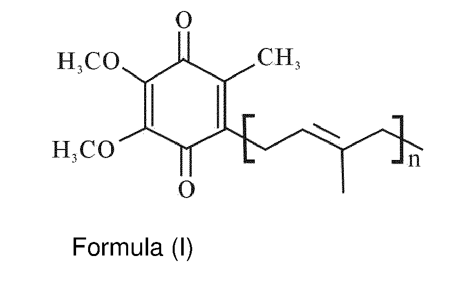

## Description

### BACKGROUND

### Field of Invention

The present invention relates to compounds. More particularly, the present invention relates to compounds and pharmaceutical compositions thereof for inhibiting mammalian tumor cell proliferation.

### Description of Related Art

According to statistics, every year 250 thousand people died from the breast tumor over world. In Taiwan for female tumor the incidence rate of breast tumor is the second highest. From 1990 to 1991 in Taiwan the number of people died from tumor is increased to 32,993, which is increased by 3.83% in one year and occupies 26.05% of the death rate. The breast tumor is one of the ten lethal tumors, and the incidence rate of breast tumor tends to increase gradually. The age of onset in Taiwan is younger than that in occident countries. Every year about 1,200 women are died from the breast tumor. Thus the breast tumor greatly threatens the health of women. The incident of cancer is closely associated with the gene and the acquired environment, and is especially associated with food and drink.

The treatment method of breast tumor includes surgical operation, radiation therapy, chemotherapy, endocrine therapy, immunization therapy and TCM (traditional Chinese medicine) therapy. The treatment method of breast tumor is determined according to the stage of the disease, the age of the patient, whether the patient is in a menopause state, the tumor size, whether some hormone receptors exist on the tumor cell.

Surgical operation and radiotherapy are used to ensure all tumor cells are totally destructed at the earliest stage of tumor. If a few tumor cells are remained or exist at other sites, it is dangerous. Thus the pharmacotherapy (or referred to as adjuvant therapy) which includes hormone therapy, chemotherapy and monoclonal antibody therapy should also be applied.

Currently the drugs for treating the breast tumor have different side effects, and thus the health condition and medical history of the patient should be taken into consideration when the drugs are used, so as to realize an optical treating effect.

### SUMMARY

In view of the above, an aspect of the present invention provides compounds of following general formula (I) for inhibiting mammalian tumor cell proliferation:

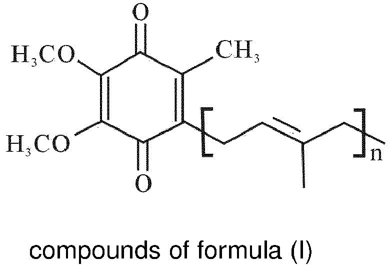

In formula (I), n is an integer from 0 to 9. The cell proliferation of breast tumor, ovarian tumor, skin tumor or liver tumor is inhibited by the compounds of the formula (I).

According to an embodiment of the present invention, the cell proliferation of the breast tumor cells which are an ERα (estrogen receptor alpha) + breast tumor cell strain or ERα- breast tumor cell strain is inhibited by the compounds of the formula (I).

Another aspect of the present invention provides pharmaceutical compositions for inhibiting mammalian tumor cell proliferation, including the compounds of formula (I) mentioned above, an extract of a processed product or natural product including the compounds of formula (I) as the main active ingredient, the pharmaceutically acceptable salts of the compounds of formula (I), the pharmaceutically acceptable solvate of compounds of formula (I) or any combination thereof.

According to an embodiment of the present invention, in the pharmaceutical compositions for inhibiting mammalian tumor cell proliferation, the processed product including the compounds of formula (I) as the main active ingredient is a fermentation liquid of Antrodia camphorata (Antrodia cinnanonea).

According to another embodiment of the present invention, the pharmaceutical compositions for inhibiting mammalian tumor cell proliferation have a form of Oral agent, injection agent or external coating agent.

A further aspect of the present invention provides an application of the compounds of formula (I). The compounds of formula (I) is used for inhibiting epithelial-mesenchymal transition (EMT) or is used to manufacture drugs for inhibiting EMT.

Still a further aspect of the present invention provides an application of the compounds of formula (I). The compounds of formula (I) is used for inhibiting metastasis of breast tumor cells or is used to manufacture drugs for inhibiting metastasis of breast tumor cells.

An aspect of the present invention provides an application of the compounds of formula (I). The compounds of formula (I) is used for inhibiting angiogenesis or is used to manufacture drugs for inhibiting angiogenesis.

A further aspect of the present invention provides an application of the compounds of formula (I). The compounds of formula (I) is used for inhibiting expression of Her2/neu or is used to manufacture drugs for inhibiting expression of Her2/neu.

Still a further aspect of the present invention provides an application of the compounds of formula (I). The compounds of formula (I) is used for regulating apoptosis/autophagy of breast tumor cells or is used to manufacture drugs for regulating apoptosis/autophagy of breast tumor cells.

In view of the above, the compounds of formula (I) of the embodiments of the present invention are applicable to manufacture ER+ or ER- drugs. The action mechanism of compounds of formula (I) for inhibiting the breast tumor includes inhibiting EMT, tumor cell metastasis, angiogenesis and regulating apoptosis/autophagy of breast tumor cells.

### BRIEF DESCRIPTION OF THE DRAWINGS

In order to make the foregoing as well as other aspects, features, advantages, and embodiments of the present invention more apparent, the accompanying drawings are described as follows:
Fig. 1A is a quantitative diagram showing the effect of compounds of formula (I) in different doses on inhibiting viabilities of human normal breast cells (MCF-10A), non-invasive breast tumor cells (MCF-7) and invasive breast tumor cells (MDA-MB-231);
Fig. 1B illustrates the effect of compounds of formula (I) on colony formation of the breast tumor cells MDA-MB-231 observed through a colony formation assay;
Fig. 2A illustrates the western blotting results of EMT-related proteins in the breast tumor cells MDA-MB-231, where the breast tumor cells MDA-MB-231 are treated with compounds of formula (I) having concentrations of 0, 0.5, 1 and 2 mM for 24 h;
Fig. 2B illustrates the western blotting results of EMT-related proteins in the breast tumor cells MCF-7, where the breast tumor cells MCF-7 are treated with compounds of formula (I) having concentrations of 0, 10, 15 and 20 µM for 24 h;
Fig. 3 illustrates a quantitative analysis diagram showing Luciferase activity of E-cadherin obtained from a primary antibody E-cadherin by an immunocytochemical analysis (ICC);
Fig. 4 illustrates photographs taken at a 200× field under a fluorescence microscope, where a primary antibody NF-κB is used and the cells are stained through the immunofluenrence staining method;
Fig. 5A illustrates the effect of compounds of formula (I) in different doses on specific protein content in breast tumor cells, which is detected by the western blotting assay;
Fig. 5B illustrates the effect of compounds of formula (I) in different doses on enzyme activities of the MMP-9, MMP2 and uPA in breast tumor cells, which is detected by the Zymography enzyme activity assay;
Fig. 6 illustrates the effect of increased treatment time of compounds of formula (I) on content of proteins ERK1/2, p38 and JNK1/2 in breast tumor cells and the protein phosphorylation reactions, which is detected by the western blotting assay;
Fig. 7 illustrates the effect of increased treatment time of compounds of formula (I) on content of proteins PI3K and Akt in breast tumor cells and the protein phosphorylation reactions, which is detected by the western blotting assay;
Fig. 8 illustrates the effect of increased treatment doses of compounds of formula (I) on the ROS content in breast tumor cells, which is detected by the reactive oxygen species method;
Fig. 9 is a quantitative diagram showing the MTT assay result of the breast tumor cells cultured and treated with compounds of formula (I);
Fig. 10 is a quantitative diagram showing the effect of compounds of formula (I) in different doses on tumor cell metastasis, which is detected by the wound scratching assay;
Fig. 11 is a diagram showing the effect of compounds of formula (I) on breast tumor cell metastasis, which is detected by the cell migration assay;
Fig. 12 illustrates the MTT assay results of human umbilical vein endothelial cells (HUVECs) when compounds of formula (I) in different doses are used;
Fig. 13 illustrates the effect of compounds of formula (I) in different doses on migration of cells stimulated by TNF-α detected by the wound scratching assay, where the cells are observed under a microscope;
Fig. 14 illustrates the effect of compounds of formula (I) on the enzyme activity of MMP-9 and MMP2 generated by cells induced and stimulated by 10ng/mL TNF-α, which is detected by the Zymography enzyme activity assay;
Fig. 15 illustrates the effect of compounds of formula (I) in different doses on cell viability of breast tumor cells MDA-MB-453;
Fig. 16 illustrates expression results of Her-2/neu-related proteins used for facilitating survival of tumor cells when compounds of formula (I) in different doses are added, which are detected by the western blotting;
Fig. 17 is a quantitative diagram showing the ROS content of breast tumor cells treated with compounds of formula (I), which is detected by the reactive oxygen species method;
Fig. 18 shows the MTT assay of breast tumor cells treated with 40 µM compounds of formula (I) under the existence of 2.5 mM antioxidant NAC;
Fig. 19 illustrates the expression results of proteins LC3B associated with cell autophagy after the breast tumor cells MCF-7 are cultured for 72 h under existence of compounds of formula (I) having different concentrations;
Fig. 20 illustrates the expression results of proteins LC3-II associated with cell autophagy after the breast tumor cells MDA-MB-231 are cultured for 24, 48 or 72 h under existence of compounds of formula (I) having different concentrations;
Fig. 21 illustrates the relationship between the tumor volume and the growth weeks of MDA-MB-231 inoculated nude mice in the control group and the group injected with compounds of formula (I);
Fig. 22 illustrates photomicrographs taken during the histopathological examination;
Fig. 23 illustrates photomicrographs of expression results of signaling-related proteins in the cells of tumor inoculated nude mice after the cells are stained through the immunochemistry staining method;
Fig. 24 illustrates quantitative results of reaction of the primary antibody E-cadherin in Fig. 23; and
Fig. 25 illustrates quantitative results of reaction of the primary antibody Vimentin in Fig. 23.

### DETAILED DESCRIPTION

According to embodiments of the present invention, compounds of the formula (I) for inhibiting tumor cell proliferation are chemically synthesized or abstracted from a natural product, a processed product or an extract thereof. According to embodiments of the present invention, the synthesized compounds of the formula (I), or the natural product, the processed product or the extract containing the compounds of the formula (I) obtained from a fermentation liquid of Antrodia camphorata have the same effect on inhibiting cell proliferation of breast tumor. Thus, in the following test examples, compounds of formula (I) having a structure where n = 0 are the subject matter of the tests. It should be understood that a mixture including the compounds of formula (I) as the main active ingredient also has the same function as the pure compounds of formula (I).

The following embodiments illustrate specific aspects of the disclosure, and those of skills can understand and implement the disclosure after reading the following embodiments. However, the scope of the disclosure is not limited to these embodiments.

### Embodiments

### 1. Compounds of formula (I) can inhibit viability of various tumor cells Test example 1: Compounds of formula (I) can affect the viability of tumor cells in different tissues

The compounds of formula (I) in the embodiments of the present invention have the capability of inhibiting various tumor cells. Half inhibit concentration (IC₅₀) of the compounds of formula (I) for inhibiting various tumor cells are illustrated in the table 1 below.

**Table 1 Analysis of the effect of the compounds of formula (I) on viability of tumor cells in different tissues**

| Cell Type | IC₅₀ (μM) |
|---|---|
| (1) Breast Tumor Cell (MDA-MB-231 cell strain) | 7.51 |
| (2) Breast Tumor Cell (MCF-7 cell strain) | 27.67 |
| (3) Breast Tumor Cell (MDA-MB-453 cell strain) | 36.68 |
| (4) Ovarian Tumor Cell (SKOV3 cell strain) | 15.32 |
| (5) Rat Skin Tumor Cell (B16F1 cell strain) | 12.81 |
| (6) Liver Tumor Cell (Huh-7 cell strain) | 15.10 |
| (7) Skin Tumor Cell (A2058 cell strain) | 23.55 |
| (8) Rat Skin Tumor Cell (B16F10 cell strain) | 20.64 |
| (9) Normal Laticiferous Cell (MCF-10A cell strain) | 24.10 |
| (10) Normal Keratinocyte Cell (HaCaT cell strain) | 35.21 |
| (11) Normal Endothelial Cell (EAhy926 cell strain) | 35.10 |
| (12) Normal Rat Macrophage Cell (RAW264.7 cell strain) | 25.54 |

It can be seen from Table 1 that the compounds of formula (I) inhibit the breast tumor cell most significantly, and the IC₅₀ of the compounds of formula (I) for inhibiting the breast tumor cell is 7.51 μM. Therefore, taking the breast tumor cell as an example, the following illustrates the effect of compounds of formula (I) on cell proliferation of the breast tumor cell.

### Test example 2-1: compounds of formula (I) inhibit viability of the breast tumor cell

The cells used in the test are human breast cells MCF-10A, and human breast tumor cells MCF-7 and MDA-MB-231. MCF-7 is an ERα (estrogen receptor alpha) + breast tumor cell strain, and MDA-MB-231 is an ERα- breast tumor cell strain.

The breast tumor cells (MDA-MB-231 and MCF-7) were commercially obtained from the bioresource collection and research center (BCRC), and were cultured in a 10% FBS-DMEM culture medium (i.e., a DMEM culture medium including 10% Fetal bovine serum (FBS)). The human breast cell MCF-10A was cultured in a 5% FBS-DMEM culture medium (including F12, insulin, hydrocortisol and epidermal growth factor). An environment of 5% CO₂ was kept in the incubator at a constant temperature of 37°C. When a certain number of cells were generated in culture, the culture medium was removed after being centrifuged for 10 min at a rate of 900 rpm. The culture medium was replaced in 24 h before the experiment, and the number of cells was counted by a hemocytometer.

The cell viability was determined by a method of transcriptional and translational assay (MTT assay). The test method for assaying cell viability refers to steps provided by Parada-Turska et al.

The breast tumor cells MDA-MB-231 were inoculated in a 24-well plate (2.5×10⁵ cells/well), and the next day after cells were adhered to the wall of the plate, the cells were washed with PBS. 1% FBS-DMEM culture medium was then added so as to synchronize cells. The next day compounds of formula (I) having different concentrations were added into the wells, and the cells were subsequently cultured in an atmosphere of 5% CO₂ at 37°C for 24 h. The cells were washed with PBS once, and 400 μl MTT agent was added in each well so as to react for 4 h. Then 400 μl 10% SDS was added in each well so as to react for 12 h. 200 μl supernatant was taken from the well and then added into a 96-well microplate, so as to measure the absorbance at 570 nm.

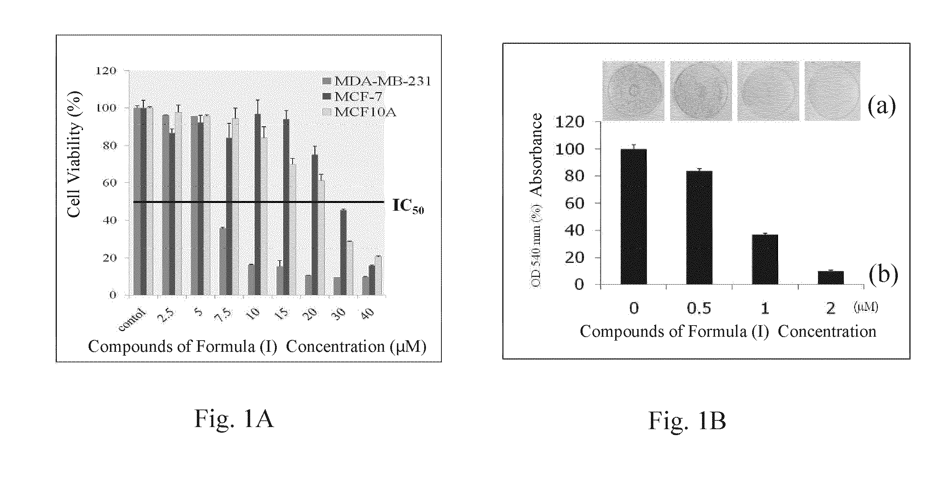

Fig. 1A is a quantitative diagram showing the effect of compounds of formula (I) in different doses on inhibiting viabilities of human normal breast cells (MCF-10A), non-invasive breast tumor cells (MCF-7) and invasive breast tumor cells (MDA-MB-231), where a data result is represented by a mean ± SD value, n = 3 and p < 0.05.

It can be seen from Fig. 1A that when compounds of formula (I) having a concentration of 0-40 μM were applied in the MCF-10A, MCF-7 and MDA-MB-231 cells for 24 h, the IC₅₀ of the compounds of formula (I) for inhibiting the normal breast cells MCF-10A was about 25 μM; the IC₅₀ of the compounds of formula (I) for inhibiting the MCF-7 was about 30 μM; and the IC₅₀ of the compounds of formula (I) for inhibiting the MDA-MB-231 was about 6.5 μM, which shows the strongest inhibiting capability.

It can be known from the results above that the compounds of formula (I) show a strongly inhibiting effect on the viability of the breast tumor cell strain MDA-MB-231.

### Test example 1-2: compounds of formula (I) inhibit colony formation of tumor cells

In order to know the effect of compounds of formula (I) on the colony formation of the breast tumor cells MDA-MB-231, the cells were observed through a colony formation assay.

Section (a) of Fig. 1B shows the colony formation, where the cells were adhered to the culture medium and react with the compounds of formula (I) having different concentrations for 24 h, and then the cells were cultured in fresh cell culture medium including 10% FBS; section (b) of Fig. 1B shows numerical values of absorbance, where the colony formed in section (a) was scanned to computer and then the colony was dissolved in 70% alcohol so as to measure the absorbance at 540 nm.

The result illustrated in the section (a) of Fig. 1B shows that the compounds of formula (I) can significantly inhibit colony formation of breast tumor cells; and the result illustrated in the section (b) shows that in the test the number of formed breast tumor cell colony was associated with the dose of compounds of formula (I).

### 2. Compounds of formula (I) inhibit epithelial-mesenchymal transition (EMT) of breast tumor cells

### Test example 2-1: compounds of formula (I) inhibit epithelial-mesenchymal transition (EMT) of breast tumor cells

Tumor metastasis is a process where the tumor cells spread from the original site to an organ tissue at a distant site through a series of steps and form a new tumor mass. Tumor cells obtain the capability of cell invasion and metastasis, which leads to a cell morphology transition from epithelial cells to mesenchymal cells, and meanwhile the original cell characteristics are changed. Thus, EMT is a transition process from epithelial cells to mesenchymal cells.

Tumor metastasis is a process of invasion start, extravasation and metastasis. During invasion, tumor cells lose adhesion between cells and obtain the capability of metastasis; and subsequently extravasated tumor cells enter the blood and lymphatic system, and finally the tumor cells travel to the target site so as to form new tumor cells.

Tumor cells use multiple signaling pathways to initiate the EMT process, which is associated with activation of multiple proteins, such as the transforming growth factor β (TGFβ), the Wnt/β-catenin, the Phosphoinositide 3-kinases (PI3K) and the Nuclear factor-κB (NF-κB). When the cells were activated by the TGF-β, transcription factors such as the Twist, the Snail, the slug and the SIP1 further repressed expression of an epithelial cell marker module E-cadherin, and increased expression of mesenchymal cell marker modules N-cadherin, Integrins and Vimentin and expression of matrix metalloproteinases (MMPs), so as to facilitate cell metastasis and invasion.

Furthermore, change of environment around tumor cells is a significant factor for determining whether tumor cells in situ metastasize. Multiple cytokines and growth factors, including the hepatocyte growth factor (HGF), the epidermal growth factor (EGF), the vascular endothelial growth factor (VEGF) and the transforming growth factor-β (TGF-β) most probably facilitate the tumor metastasis. Particularly, these grow factors can activate expression of related proteins such as N-cadherin, vimentin, fibronectin, snail, slug, twist, MMP-2, MMP-3 or MMP-9 through signaling pathways such as PKC, PI3/AKT and HGFR-cMet so as to facilitate the tumor metastasis, and relatively repress expression of related proteins such as E-cadherin, desmoplakin, occluding and the cell adhesion molecule (CAM).

Thus the following illustrates a western blotting of cell marker proteins, so as to research the action mechanism of compounds of formula (I) for inhibiting tumor cells.

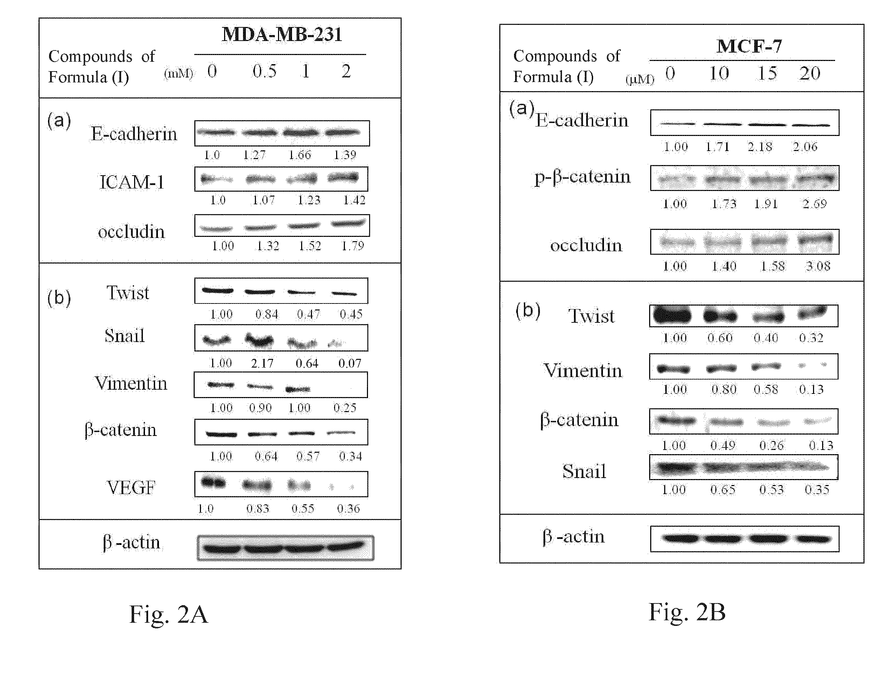

Referring to Figs. 2A and 2B, the western blotting was used to detect effect of compounds of formula (I) in different doses on specific protein content in breast tumor cells.

Fig. 2A illustrates the expression results of EMT-related proteins in breast tumor cells MDA-MB-231 after the breast tumor cells MDA-MB-231 have been cultured under compounds of formula (I) each having concentrations of 0, 0.5, 1 and 2 mM for 24 h; and Fig. 2B illustrates the expression results of EMT-related proteins in breast tumor cells MCF-7 after the breast tumor cells MCF-7 have been cultured under compounds of formula (I) each having concentrations of 0, 10, 15 and 20 μM for 24 h.

The steps of western blotting include: washing cells with PBS twice at 4°C; adding a lysis buffer and rubbing the cells by a rubbr policeman so as to lyse cells; putting the lysed cell solution into a 1.5 ml microcentrifuge tube and shaking for 1 min; centrifuging at 12000 rpm for 10 min and taking the supernatant; measuring concentration of the protein through a Bio-Rad protein assay kit; and storing the remained extracted protein solution at -70°C; taking 100 μg protein solution and adding a SDS/protein loading buffer having the same volume, where the SDS/protein loading buffer has a concentration two times larger than the protein solution; boiling the mixture of the protein solution and the SDS/protein loading buffer at 100°C for 10 min; cooling and loading the protein samples in each hole of gel; adjusting the voltage to 100 V and running the protein samples on the gel until the tracking dye in the sample buffer reaches the bottom of the gel; and subsequently transferring the protein from the gel using electrophoresis. A PVDF membrane was soaked in methanol until the paper was totally wet. The paper was arranged in a transfer tank placed in a 4°C ice box, and the protein transfer was performed at a current of 600 VA for 4 h. The PVDF membrane was then taken out, placed in a PBS blocking solution including 1% BSA and shaken for 2 h. Then the PVDF membrane was placed in a blocking solution including a primary antibody and slowly shaken at 100 rpm for 1 h at the room temperature. Subsequently the PVDF membrane was washed with PBST for third times, each time for 3-5 min. According to different first antibodies and different dilution ratios, the PVDF membrane was shaken for 2 h on a horizontal rotator and then washed with PBST for three times. Then luminescence image equipment was used, where the PVDF was placed onto a black metal plate by a plastic pincer and the face including protein was upwards, a 1:1 mixed Chemiluminesent Substrate was added into the membrane and the exposure time depends on the fluorescence intensity of antibodies.

In Fig. 2A the section (a) illustrates the expression results of proteins used for inhibiting the tumor cell proliferation in breast tumor cells MDA-MB-231, such as the E-cadherin, the occludin and the ICAM; the section (b) illustrates the expression results of proteins used for facilitating the tumor cell proliferation in breast tumor cells MDA-MB-231, such as the Twist, the Snail, the vimentin, the β-catenin and the VEGF. β-actin was an internal control.

The results of section (a) in Fig. 2A show that along with dose increasing of the compounds of formula (I), the expression amount of proteins used for inhibiting the tumor cell proliferation in the breast tumor cells MDA-MB-231, such as the E-cadherin, the occludin and the ICAM was increased, which presents a dosage association relationship. On the contrary, The results of section (b) in Fig. 2A show that along with dose increasing of the compounds of formula (I), the expression amount of proteins used for facilitating the tumor cell proliferation in the breast tumor cells MDA-MB-231 was decreased.

Referring to Fig. 2B, the section (a) shows the expression results of proteins used for inhibiting the tumor cell proliferation in breast tumor cells MCF-7, such as the E-cadherin, the p-β-catenin and the occludin; the section (b) shows the expression results of proteins used for facilitating the tumor cell proliferation in breast tumor cells MCF-7, such as the Twist, the Snail, the vimentin and the β-catenin. β-actin is an internal control.

Fig. 2B illustrates a similar result as Fig. 2A. The results of section (a) in Fig. 2B show that along with dose increasing of the compounds of formula (I), the expression amount of proteins in the breast tumor cells MCF-7 used for facilitating the tumor cell proliferation, such as the E-cadherin, the p-β-catenin and the occludin was increased, which presents a dosage association relationship. On the contrary, The results of section (b) in Fig. 2B show that along with dose increasing of the compounds of formula (I), the expression amount of proteins in the breast tumor cells MCF-7 used for facilitating the tumor cell proliferation was decreased.

Thus, compounds of formula (I) have significant inhibiting effect on EMT, and now referring to some documents, when E-cadherin was over-expressed in invasive tumor cells, the invasive tumor cells became non-invasive, which can facilitate the tumor cell proliferation.

### Test example 2-2: compounds of formula (I) improves expression of E-cadherin in breast tumor cells

According to the assay result of western blotting, compounds of formula (I) had an improving effect on expression of E-cadherin in breast tumor cells and an inhibiting effect on expression of Vimentin in breast tumor cells. Thus, taking the MDA-MB-231 cell as an example, the expression results of E-cadherin and Vimentin in the cell were observed.

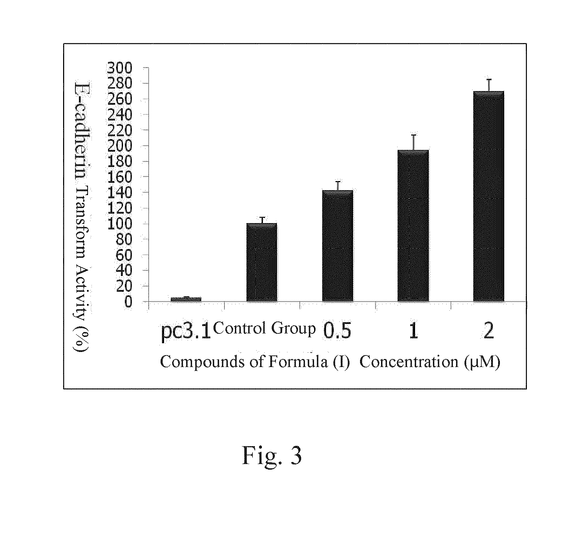

Fig. 3 is a quantitative analysis diagram showing Luciferase activity of E-cadherin obtained from a primary antibody E-cadherin by an immunocytochemical analysis (ICC). In Fig. 3, a transformed cell strain only including an empty vector (pC3.1) is the negative control, and a cell strain including no compounds of formula (I) is the background control, the translational activity is defined as 100%.

ICC includes embedding the tumor cells in paraffin and slicing the embedded cells into slides having a thickness of 5 mm by a slicer, deparaffinizing, rehydrating and renaturating, picking the slide out and putting the slide into the primary antibody E-cadherin; reacting for 1.5 h and washing the slide for three times with PBS, reacting with the secondary antibody for 30 min and washing the slide for three times with PBS, staining the slide with 3',3'-diaminobenzendine (DAB) at a ratio of 1:30, and the slide was mounted with Entellanz.

The results of Fig. 3 show that along with the dose increasing of compounds of formula (I), the expression amount of E-cadherin in the cytoplasm of MDA-MB-231 was increased; and the compounds of formula (I) inhibit expression of vimentin in MDA-MB-231 cells.

### Test example 2-3: compounds of formula (I) improves expression of NFκB in breast tumor cells

As described above, the compounds of formula (I) can significantly improve expression of epithelial marker module E-cadherin and inhibit expression of mesenchymal marker module Vimentin in breast tumor cells. Thus, by researching expression of activation factors at upstream and downstream of the signaling pathway, the related mechanism for inhibiting tumor cell proliferation is further understood.

Since during tumor cell metastasis, the transcriptional activity of nuclear factor-κB (NF-κB) is a very important key factor, and NF-κB regulates expression of proteins associated with cell metastasis, the effect of compounds of formula (I) on EMT signaling is further understood through the expression results of NF-κB in the cells.

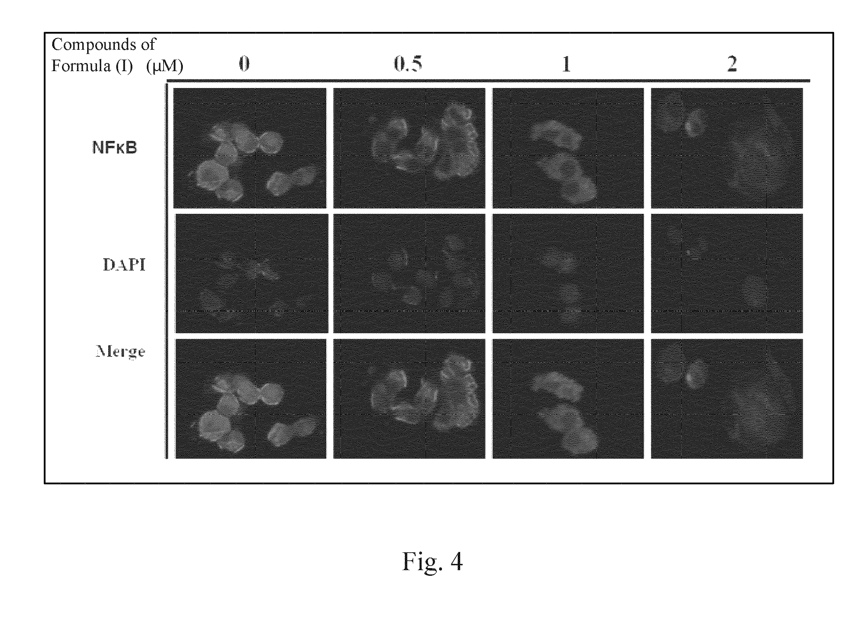

Fig. 4 illustrates photographs taken at a 200× field under a fluorescence microscope, where a primary antibody NF-κB was used and the cells were stained through the immunofluenrence staining method.

In the immunofluenrence staining, compounds of formula (I) in different concentrations were used to stimulate MDA-MB-231 cells, and a primary antibody NF-κB (with a dilution ratio of 1:250) was added, and then the cells were cultured at an atmosphere of 5% CO₂ for 2 h at 37°C. Subsequently a fluorescence secondary antibody (with a dilution ratio of 1:200) was added, and finally DAPI (with a dilution ratio of 1:1000) was added. The cells were stored with protect from light at the room temperature for 5 min. The cells were observed under the fluorescence microscope and a photograph (200x) of the cells was taken. Finally, the cells were mounted with the mounting gel.

The results of Fig. 4 show that the compounds of formula (I) can inhibit expression of NF-κB, and may be associated with inhibiting expression of proteins related to metastasis.

### 3. Compounds of formula (I) inhibit metastasis of breast tumor cells Test example 3-1: compounds of formula (I) inhibit expression of MMPs in breast tumor cells

During tumor cell metastasis, the extracellular matrix (ECM) was often degraded mainly by matrix metalloproteinase (MMPs) and a urokinase-type plasminogen activator (uPA) facilitating activation of MMPs. Breast tumor cells can secrete a lot of MMPs. Many researches point out that the MMP-2 and MMP-9 play very important roles in angiogenesis and the tumor cell metastasis process. Furthermore, expression of MMPs inhibitions TIMP-1, TIMP-2 can repress activity of the MMPs, and the plasminogen activator inhibition (PAI) PAI-1 can repress activity of uPA, so as to inhibit invasion and metastasis of tumor cells.

Thus, the following illustrates a western blotting of cell proteins associated with the angiogenesis and metastasis of tumor cells in situ, so as to illustrate the action mechanism of compounds of formula (I) for inhibiting tumor cell metastasis.

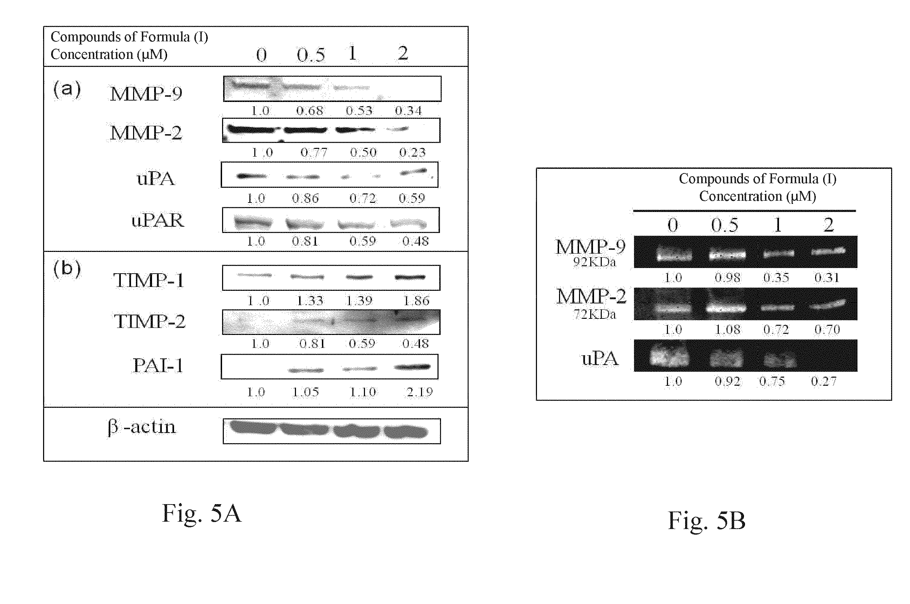

Referring to Fig. 5, the western blotting was used to detect effect of compounds of formula (I) in different doses on specific protein content in breast tumor cells. Fig. 5 shows the expression results of proteins associated with cell metastasis in breast tumor cells after the cells were cultured in compounds of formula (I) each having a concentration of 0, 0.5, 1 and 2 μM for 24 h. The steps of western blotting were the same as that shown in the test example 2-2, and thus it is not illustrated any further.

In Fig. 5A, section (a) shows the expression results of MMP-2, MMP-9, uPA and uPAR in the breast tumor cells; section (b) shows the expression results of TIMP-1, TIMP-2 and PAI-1 in breast tumor cells. β-actin is an internal control.

The results of section (a) in Fig. 5A show that along with the dose increasing of compounds of formula (I), the expression of the MMP-2, the MMP-9, the uPA and the uPAR in breast tumor cells was inhibited, which presents a dose association relationship. In contrary, section (b) in Fig. 5A show that along with the dose increasing of compounds of formula (I), the expression amount of the TIMP-1, the TIMP-2 and the PAI-1 was increased.

It can be seen from the results above that compounds of formula (I) can prevent the MMPs from activating by facilitating expression of MMP inhibitions and inhibiting function of the activator uPA of MMP, so as to inhibit the activity of MMPs and inhibit invasion and metastasis of tumor cells.

Referring to Fig. 5B, the Zymography enzyme activity assay was used to detect effect of compounds of formula (I) in different doses on enzyme activities of the MMP-9, MMP2 and uPA in breast tumor cells.

In the Zymography enzyme activity assay, the cells (1×10⁶) were planted in a 6-well plate. The next day after the cells were adhered to the wall, the cells were cultured in a serum free DMEM culture medium at an atmosphere of 5% CO₂ in an incubator at 37°C for 24 h. Compounds of formula (I) in different doses were added into the DMEM culture medium. The cells were cultured in the culture medium for 2 h and then washed with PBS. The cell culture medium was putted into a microcentrifuge tube and centrifuged for 10 min at 1500 rpm. The supernatant was taken to perform a protein gel electrophoresis (SDS-PAGE), so as to separate the proteins. Afterwards, the Gelatin or Casein was regarded as the substrate to analysis the enzyme activity of the proteins.

The results of Fig. 5B show that with the dose increasing of compounds of formula (I), enzyme activities of the MMP-2, the MMP-9 and the uPA used for facilitating angiogenesis and tumor cell metastasis in breast tumor cells were decreased.

### Test example 3-2: compounds of formula (I) inhibit protein phosphorylation reactions used for tumor cell signaling

In tumor cells, serine/threonine kinases-mitogen activated protein kinases (MAPKs) play a role in signaling pathway of activating the MMPs and uPA and in the signal transduction process of cell apoptosis. MAPKs include ERK 1/2 (extracellar signal-regluated kinase), JNK/SAPK (c-Jun NH2-terminal kinase) and p38. MAPKs have multiple members and multiple reaction pathways. The signaling process was performed by a series of protein phosphorylation reactions.

The phosphorylated MAPK-related proteins can transfer signals in cells. The effects of MAPKs on physiological functions of cells include inflammination, apoptosis, oncogene transformation, tumor cell metastasis, so as to down-regulate transcription factors in cell nuclei and affect expression of various proteins including proteins associated with metastasis. The researches in different cells show that the ERK1/2, P38 and JNK pathways can regulate the expression of MMPs and uPA. For example, when the phosphorylation of p38 was inhibited, the expression amount of TIMP-2 was increased, and the metastasis capability of tumor cells was inhibited, and meanwhile JNK played an important role in inhibiting tumor cell metastasis. Thus inhibiting the MAPKs pathway was associated with inhibiting the angiogenesis, the cell proliferation and tumor cell metastasis.

Thus, the following illustrates a western blotting of cell proteins associated with the angiogenesis, the cell proliferation and the tumor cell metastasis, so as to illustrate the action mechanism of compounds of formula (I) for inhibiting tumor cell metastasis.

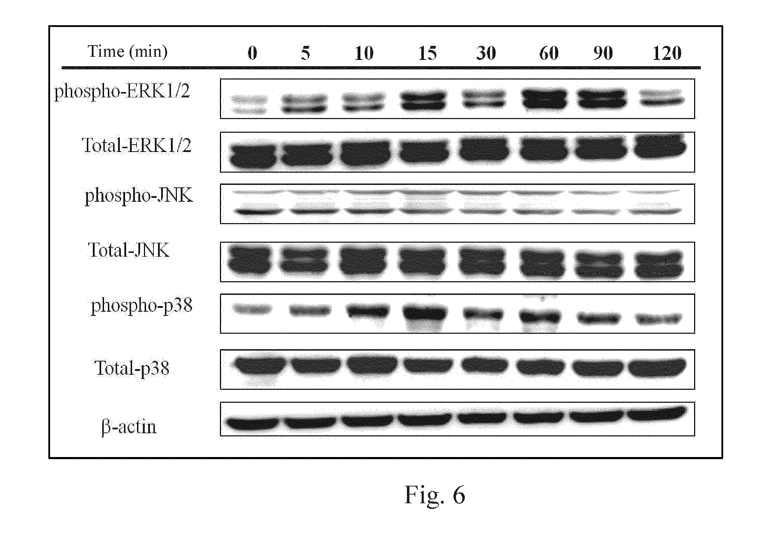

Referring to Fig. 6, the western blotting was used to detect the effect of increased treatment time of compounds of formula (I) on content of proteins ERK1/2, p38 and JNK1/2 in breast tumor cells and the protein phosphorylation reactions. The western blotting was performed after the breast tumor cells were treated with 2 μM compounds of formula (I) for 0-120 min. Analysis steps of the western blotting are the same as that shown in the test example 2-2, and is not illustrated any further.

The results of Fig. 6 show that along with the increasing treatment time of compounds of formula (I), protein expression of ERK1/2, p38 and JNK1/2 was not affected, but the phosphorylation thereof was probably inhibited. Thus, the signals were prevented from entering the cell nuclei and regulating the transcription factors, so as to inhibit expression of enzymes used for facilitating angiogenesis and tumor cell metastasis.

### Test example 3-3: the effect of compounds of formula (I) on signaling of tumor cells

The tumor-related proteins PI3K and Akt which are activated by the phosphorylation can regulate multiple reactions in the cells, such as cell proliferation, survival, inflammination and migration. When the growth factor receptor was activated by PI3K/Akt, the activation of downstream proteins was initiated, so as to facilitate survival of tumor cells.

Thus, the following illustrates a western blotting performed on the PI3K and Akt in cells, so as to illustrate the action mechanism of compounds of formula (I) for inhibiting tumor cell metastasis.

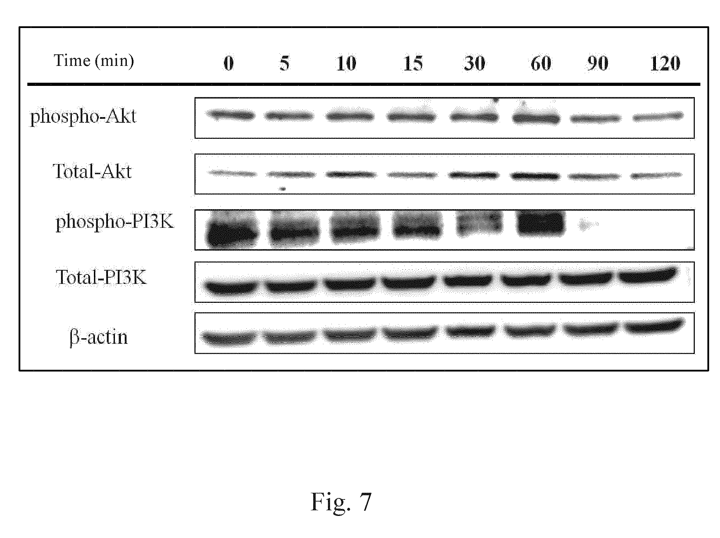

Referring to Fig. 7, the western blotting was used to detect the effect of increased treatment time of compounds of formula (I) on content of proteins PI3K and Akt in breast tumor cells and the protein phosphorylation reactions. The western blotting was performed after the breast tumor cells were treated with 2 μM compounds of formula (I) for 0-120 min. Analysis steps of the western blotting are the same as that shown in the test example 2-2, and is not illustrated any further.

The results of Fig. 7 show that along with the increasing treatment time of compounds of formula (I), protein expression of PI3K and Akt was not affected, but the phosphorylation thereof was probably inhibited. Thus, the signals are prevented from entering the cell nuclei and regulating the transcription factors, so as to affect proliferation, survival, inflammination and migration of tumor cells.

### Test example 3-4: compounds of formula (I) affect tumor cell proliferation by generating reactive oxygen

Reactive oxygen species (ROS) are modules generated by metabolism in cells of organisms under environmental stimulus, such as O⁻², ^{.}OH and H₂O₂. The possible mechanism of free radicals for causing apoptosis include that the free radicals changed DNA so as to induce the apoptosis, and the protein-free radical interaction caused the protein denaturation. Recent years many researches pointed out that the ROS played an important signaling role in normal cells.

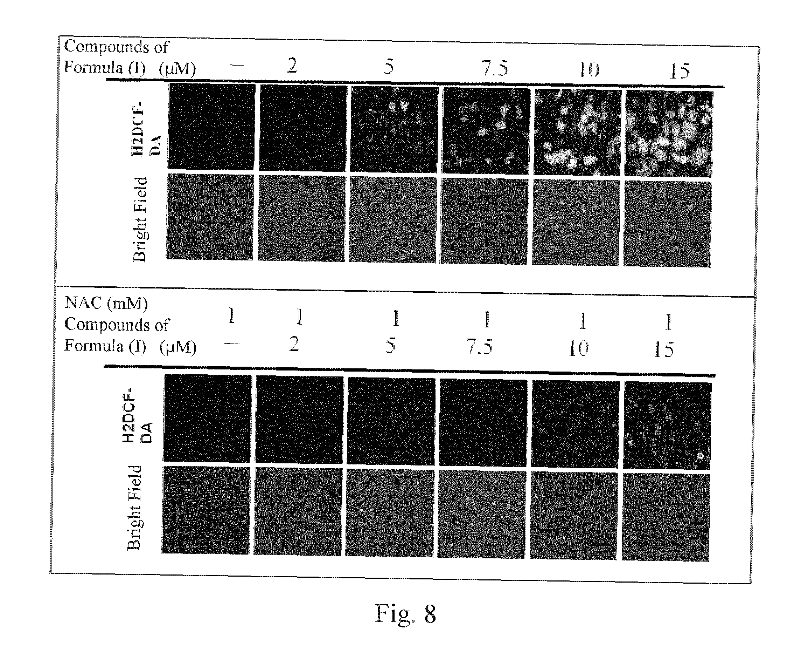

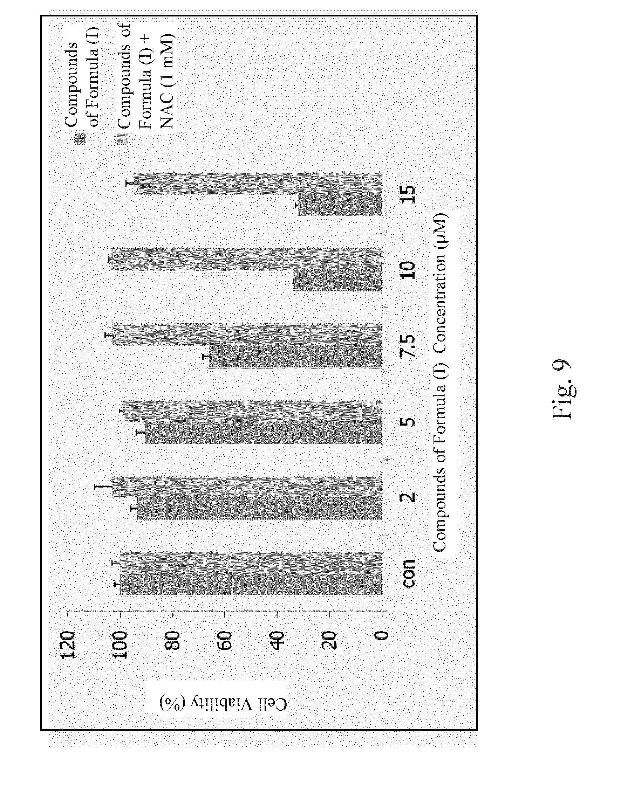

Referring both Figs. 8 and 9, Fig. 8 illustrates the result effect of increased treatment doses of compounds of formula (I) on ROS content in breast tumor cells, where the result was detected by a reactive oxygen species method; and Fig. 9 illustrates the result of MTT assay after the breast tumor cells were cultured through a same method mentioned above.

In Fig. 8, the reactive oxygen species method was used to detect the effect of increased treatment doses of compounds of formula (I) on the ROS content in breast tumor cells, and a photograph was taken at a 200× field under the fluorescence microscope. In the intracellular reactive oxygen species method, the cells were planted in a 24-well plate (5×10⁴ cells/well). The next day after the cells were adhered to the wall, the cells were cultured in a serum free DMEM culture medium at an atmosphere of 5% CO₂ in an incubator at 37°C for 12 h. Compounds of formula (I) were added into the 1% FBS-DMEM culture medium, and the cells were cultured in the culture medium for 2 h. The cells were then washed with PBS. 100 ng/mL LPS was added to react with the cells, and then the cells were washed with PBS. Culture medium including 10 μM fluorescent probe (DCFH2-DA) was supplied and then the cells were cultured for 30 min at 37°C. The cells were washed with PBS for three times so as to remove the residual stain. Finally the cells were lysed using 0.25% Tritox-100. The absorbance was measured by a fluorescence spectrometer, where the excitation wavelength was set as 485 ± 20 nm, and the scattering wavelength was set as 530 ± 25 nm.

The results of section (a) in Fig. 8 show that along with the increasing treatment doses of compounds of formula (I), the content of reactive oxygen species in the breast tumor cells MDA-MB-231 was increased accordingly, which may lead to apoptosis of tumor cells; and the results of section (b) show that when compounds of formula (I) and the antioxidant NAC were added at the same time, the increasing tend of reactive oxygen species in tumor cells MDA-MB-231 were not obvious.

Fig. 9 separately illustrates the viabilities of cells treated with compounds of formula (I) in different doses, and whether the viabilities of cells were recovered when the cells were treated with both compounds of formula (I) and the antioxidant NAC, so as to illustrate the effect of increased reactive oxygen species caused by the compounds of formula (I) on the tumor cell proliferation. The test method for measuring the cell viability refers to steps described in test example 2-1. The breast tumor cells MDA-MD-231 were treated with compounds of formula (I) of 0, 2, 5, 7.5, 10 and 15 mM for 24 h, and then the effect of compounds of formula (I) on viability of breast tumor cells MDA-MD-231 was observed. Fig. 9 is a quantitative diagram, the data result was represented by mean ± SD values, where n = 3.

The results of Fig. 9 show a similar result as Fig. 8. When the cells MDA-MB-231 were treated with compounds of formula (I) of 2-15 μM, along with the increasing treatment concentration of compounds of formula (I), the cell viability was significantly decreased; in contrary, in the test group where compounds of formula (I) and the antioxidant NAC were both added, the cell viability was not influenced.

In view of the results above, the compounds of formula (I) facilitated apoptosis of tumor cells by generating reactive oxygen species.

### Test example 3-5: compounds of formula (I) inhibit migration of tumor cells

During metastatic of tumor cells, if the tumor cells were highly metastatic cell strains, the connection between cells were damaged and the cells themselves released multiple protein decomposing enzymes so as to facilitate degradation of ECM, which increased the migration probability of tumor cells and caused the metastasis of tumor cells.

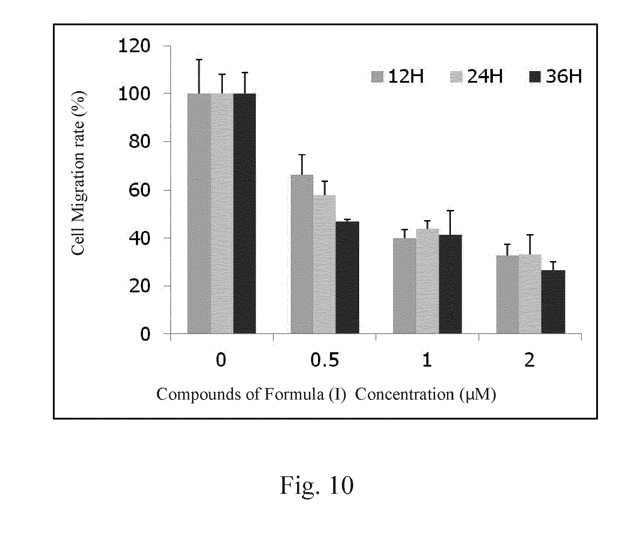

Referring to Fig. 10, the wound scratching assay was performed to analysis effect of compounds of formula (I) in different doses on tumor cell metastasis.

In the wound scratching assay for observing cell migration, the cells MDA-MB-231 (3×10⁵) were planted in a 12-well plate, and the next day the cells were washed with PBS twice. The original culture medium then was replaced by 1% FBS cell culture medium. The next day a space was scratched at the bottom of the well. The cell culture medium was removed and the cells were washed with PBS twice. 1% FBS cell culture medium and compounds of formula (I) of different concentrations were added and photographs were taken after 0, 12, 24 and 36 h so as to observe migration of cells. After the reaction, the cells were fixed for 30 min using 75% ice alcohol. After the alchol was completely volatilized, the cells were stained with Giemsa Stain. The migration number of cells was counted in three random fields (200×) under the microscope (Lin et al., 2008). In the wound scratching assay, the group including no compounds of formula (I) was regarded as the control group, and the groups where 0.5, 1 and 2 μM compounds of formula (I) were added were regarded as test groups. After the breast tumor cells were treated for 0, 12, 24 and 36 h, the quantitative result of effect of compounds of formula (I) on metastasis of breast tumor cells MDA-MD-231 was observed, and the data results were represented by mean ± SD values, where n = 3.

As shown in Fig. 10, when the number of migrated cells were counted, under compounds of formula (I) of a low concentration (0.5 μM), in 12-24 h about 60-70% breast tumor cells were migrated, and when the compounds of formula (I) had a concentration above 1 μM, the migration capability of breast tumor cells were significantly inhibited.

### Test example 3-6: compounds of formula (I) inhibit invasion of tumor cells

When malignant tumor cells prepared to migrate, the tumor cells invaded into the basement membrane of tissues by damaging the extracellular matrix (ECM). This process was referred to as invasion, where the invasion capability of tumor cells was facilitated by participating of metalloproteinase MMPs.

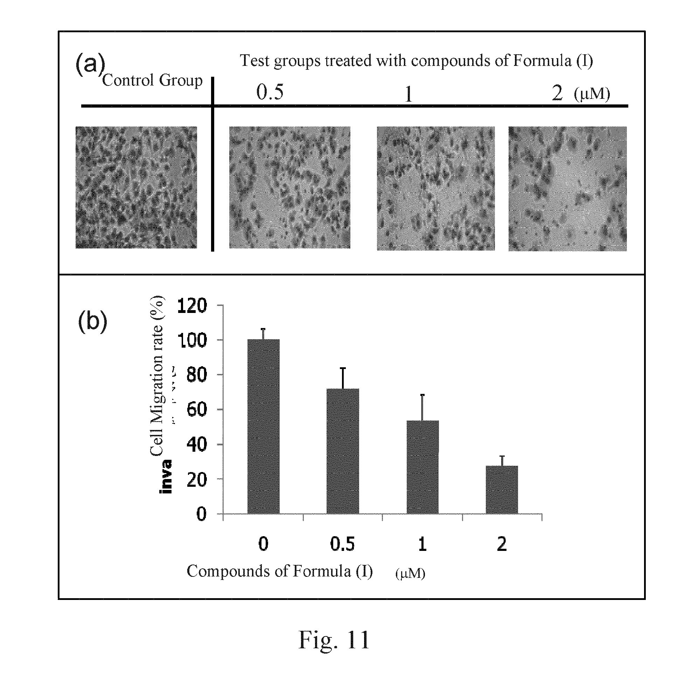

Referring to Fig. 11, section (a) shows the effect of compounds of formula (I) on metastasis of breast tumor cells observed through a transwell invasion assay; section (b) is a quantitative diagram of the results in section (a), and data results were represented by mean ± SD values, where n = 3.

In the cell migration test, the Matrigel which was a component of ECM was used to imitate the environment between cells. When the cells in a BD Matrigel™ Invasion Chamber were stimulated by growth factors in the Matrigel matrix, the cells secreted some matrix proteolytic enzymes to degrade the Matrigel, so as to facilitate migration of cells. If the cells had strong migration capability, the cells can be observed on the filter membrane after staining.

In the cell migration test, 800 μl 10% FBS-DMEM was added into the transwell as a chemoattractant, and then 500 μl cells (1×10⁵) were planted in the transwell. DMEM culture medium including no FBS and compounds of formula (I) of 0.5, 1 and 2 μM were added. The cells were then cultured at an atmosphere of 5% CO₂ in an incubator at 37°C for 20 h. Subsequently the cells were stained with Giemsa for 15 min, and observed by taking photographs at a 200× field of an optical microscope. In the test each time each group was represented by a medium value of three repeat transwells.

In the quantitative method of cell migration test, steps included randomly choosing 9 fields at the lower surface of the transwell membrane, counting the total cell number in each field; regarding the medium value of cell number in the 9 fields as an index of chemotaxis migration of breast tumor cells in each transwell, and each group of each test was represented by the medium value of 3 repeat transwells.

It can be obviously seen from the cells migration shown in the photomicrographs in section (a) of Fig. 11 that along with the increasing concentration of compounds of formula (I), the migration of tumor cells is significantly reduced. MDA-MB-231 is a highly metastasis human breast tumor cell strain. Thus in the photographs in section (a) of Fig. 11, it can be seen that in the control group many cells were attracted by chemoattractant (the 10% FBS cell culture medium) and migrated from the Matrigel which imitates the ECM to the filter membrane. In the test groups where compounds of formula (I) of 0.5, 1 and 2 μM were added, with the increasing concentration of compounds of formula (I), the migration of breast tumor cells were significantly decreased.

Furthermore, as shown in section (b) of Fig. 11, from the counted number of migrated cells, it can be seen that the migration capability of cells were reduced along with the increasing concentration of compounds of formula (I).

### 4. Compounds of formula (I) inhibit angiogenesis

In view of the test examples above, it can be seen that compounds of formula (I) affected phosphorylation reactions of ERK1/2, p38 and JNK1/2 in the MAPK pathway. Currently it has been known that the MAPKs may be associated with inhibiting cell angiogenesis, proliferation and the tumor cell metastasis. The breast tumor cells MDA-MB-231 can induce expression of MMP-9 and AP-1 through TNF-α. After inhibitions ERK or P38 was added, the expression of MMP-9 induced by the TNF-α was inhibited (Kim et al., 2008).

Thus, the following further illustrates whether the compounds of formula (I) have an effect on the TNF-α and inhibit the induced expression of MMP-9.

### Test example 4-1: compounds of formula (I) inhibit expression of MMP induced by TNF-α

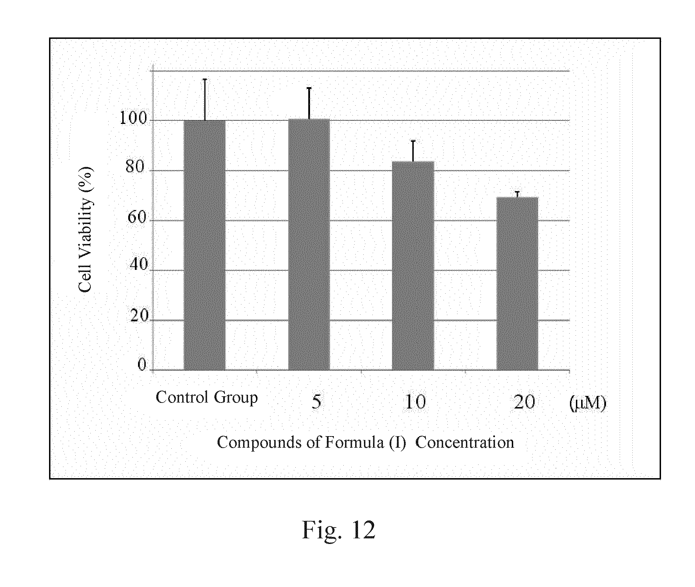

Fig. 12 illustrates the MTT assay of human umbilical vein endothelial cells (HUVECs) when compounds of formula (I) in different doses were used.

In the MTT assay, the umbilical vein endothelial cells (EAhy926 cell strain) was planted 80 percent full and cultured in a 1×HAT culture medium including 15% FBS-DMEM, 1% glutamine and 1% PSN (used for screening cells having the characteristic of cell fusion). When a certain number of cells were generated in culture, the culture medium was removed after being centrifuged for 10 min at a rate of 900 rpm. The culture medium was replaced in 24 h before the experiment, and the number of cells was counted by a hemocytometer. Then the cells (5×10⁴) were planted in a 24-well plate. The next day after the cells were adhered to the wall, the cells were treated with compounds of formula (I) of 5, 10 and 20 μM for 1 h. The cells including no compounds of formula (I) were regarded as a control group. The cells were washed with PBS twice and stimulated with 10 ng/mL TNF-α/TGF, and then washed with PBS again. The cells in each hole were treated with 400 μL MTT agent for 2 h. The supernatant was taken and putted into a 96-well microplate, and then the absorbance was measured at a wavelength of 570 nm.

The results of Fig. 12 show that when the added compounds of formula (I) had a concentration above 10 μM, the cell viability was significantly decreased.

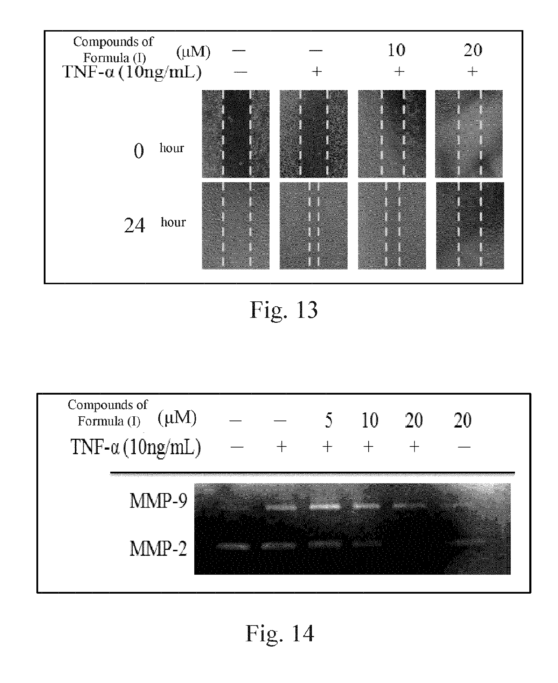

Fig. 13 illustrates the effect of compounds of formula (I) in different doses on migration of cells stimulated by 10 ng/mL TNF-α detected by the wound scratching assay, where the cells were observed under a microscope.

The method for observing cell migration was as described in the test example 3-5. In the wound scratching assay, the cells including no compounds of formula (I) and not stimulated by TNF-α were regarded as the control group, and the cells in the control group had no migration capability. The cells in the test groups were stimulated by 10 ng/mL TNF-α, and in the test groups when the cells including no compounds of formula (I) and the cells including 10 and 20 μM compounds of formula (I) were treated for 24 h, the effect of compounds of formula (I) on migration of breast tumor cells MDA-MD-231 was observed.

From the cell migration observed in the photomicrographs of Fig. 13, it can be seen obviously that in the test groups where no compounds of formula (I) was added, due to the stimulation of TNF-α, the epidermal cell has a strong migration capability, and the space scratched between cells was becoming smaller; and in the test groups where 10 and 20 μM compounds of formula (I) was added, with the increasing concentration of compounds of formula (I), the cell migration was not obvious.

It can be seen from Fig. 13 that compounds of formula (I) can counteract the stimulation effect of TNF-α on cells. Thus, it was further tested whether the compounds of formula (I) can affect the inducing effect of TNF-α on MMPs.

Referring to Fig. 14, the Zymography enzyme activity assay was performed to detect the effect of compounds of formula (I) on the enzyme activity of MMP-9 and MMP2 generated by cells induced and stimulated by 10 ng/mL TNF-α. The Zymography enzyme activity assay was described as the test example 4-1.

The cells including no compounds of formula (I) and not stimulated by TNF-α was regarded as a control group, and the cells in the control group had no enzyme activity. Cells in the test groups included no compounds of formula (I). The cells in the test groups were treated with compounds of formula (I) of 5, 10 and 20 μM so as to detect whether the compounds of formula (I) can affect the inducing effect of TNF-α on the MMPs.

It can be obviously seen from the results of enzyme activity assay in Fig. 14 that in the test groups where compounds of formula (I) were not added, the MMP-9 and MMP2 have enzyme activities; and in the test groups where 20 μM compounds of formula (I) were added, the MMP2 has no enzyme activity. Thus, the compounds of formula (I) can inhibit the inducing effect of TNF-α on MMPs.

According to the results shown in Figs. 12, 13 and 14, compounds of formula (I) can inhibit the protein activity of MMPs induced by the TNF-α, especially inhibiting the enzyme activity of MMP-2 and MMP-9 which can facilitate angiogenesis and tumor cell metastasis.

### 5. Compounds of formula (I) can inhibit expression of Her2/neu

Proto-oncogene HER-2 was positioned in the chromosome 17q21, and was associated with anti-apoptosis, the Akt pathway and the MAPK. HER-2 had an intrinsic tyrosine kinase activity and participated in regulating of cell growth, proliferation and differentiation. High expression of Her-2/neu mRNA improved the proliferation capability of tumor cells, which caused cell-cycle dysregulation and damaged DNA caused by untimely repair, thereby leading to proliferation of atypical cells.

Taking the breast tumor cells MDA-MB-453 as an example, the following illustrates the effect of compounds of formula (I) on the expression of Her-2/neu.

### Test example 5-1: compounds of formula (I) inhibit expression of Her2/neu and downstream genes

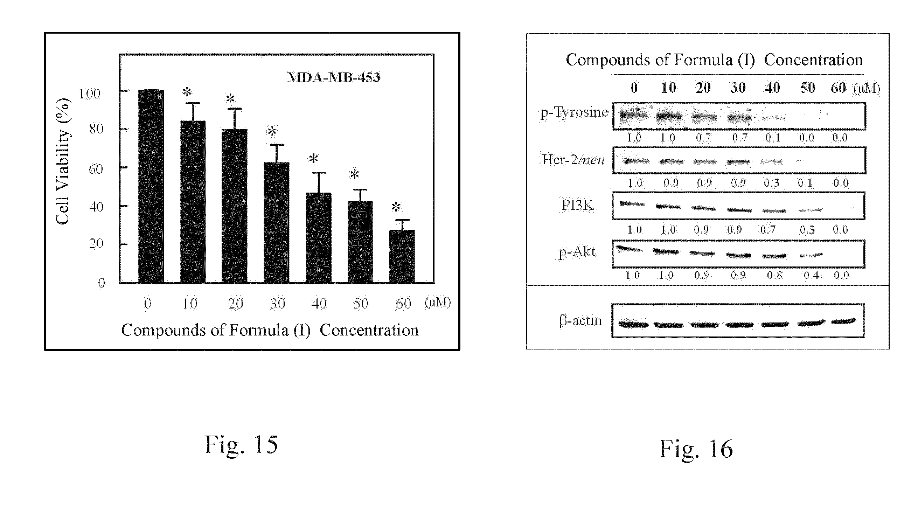

Fig. 15 illustrates the effect of compounds of formula (I) in different doses on cell viability of breast tumor cells MDA-MB-453.

In the MTT assay, the MDA-MB-453 cells were cultured in a culture medium including 5% Fetal bovine serum-DMEM F12/insulin/hydrocortisol/epidermal growth factor. Then the cells were cultured at an atmosphere of 5% CO₂ in an incubator at a constant temperature of 37°C. When a certain number of cells were generated in culture, the culture medium was removed after being centrifuged at 900 rmp for 10 min. The culture medium was replaced in 24 h before the experiment, and the number of cells was counted by a hemocytometer.

The results of Fig. 15 show that along with the increasing concentration of the added compounds of formula (I), the viability of breast tumor cells was inhibited significantly.

Fig. 16 illustrates expression results of Her-2/neu-related proteins used for facilitating survival of tumor cells when compounds of formula (I) in different doses were added, which were detected by the western blotting. The breast tumor cells were each treated with 10-60 μM compounds of formula (I), and then the western blotting of the cells was performed. Analysis steps of the western blotting are the same as that shown in the test example 2-2, and is not illustrated any further.

The results of Fig. 16 show that along with the increasing treatment concentration of compounds of formula (I), the expression amount of p-Tyrosine activated by Her-2/neu was significantly decreased, which was consistent with the expression amount of Her-2/neu. Moreover, the protein expression of PI3K and Akt was also inhibited by compounds of formula (I) in high concentration, so as to inhibit the downstream signaling, thereby affecting the growth, survival, inflammination and metastasis of breast tumor cells.

According to the results of Fig. 16, it can be seen that after the breast tumor cells were treated with 40 μM compounds of formula (I), the expression of Her-2/neu and p-Tyrosine was totally inhibited. Thus in the following the cells were treated with 40 μM compounds of formula (I) and then the ROS content of cells was detected. The intracellular reactive oxygen species method is as shown in the test example 3-4.

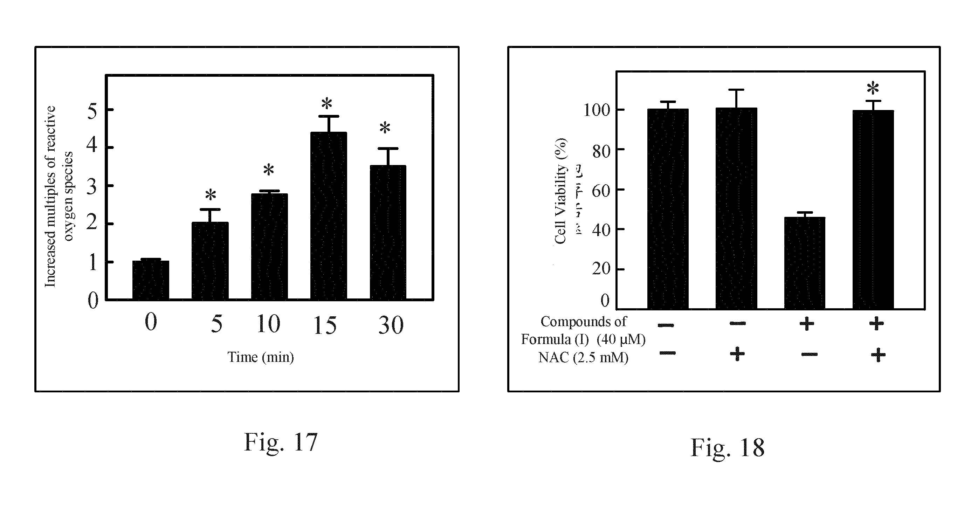

Fig. 17 is a quantitative diagram showing the effect of increasing treatment time on the ROS content of breast tumor cells when the reactive oxygen species method was performed to detect the breast tumor cells treated with 40 μM compounds of formula (I). The result data of the experiment was represented by medium value ± standard deviation (MEAN ± SD) (n = 3). A SPSS system was used to perform the analysis of variance (ANOVA), and the Duncan's multiple range test was used to perform a difference comparison between groups, where P < 0.05 represents a significant difference.

The results of the reactive oxygen species method show that compared with the control group, in the test group the content of oxygen species in the breast tumor cells treated with compounds of formula (I) is higher, which may lead to apoptosis of tumor cells.

The quantitative results of Fig. 17 show that compared with the control group where compounds of formula (I) were not added, in the test group treated with compounds of formula (I) the increased amount of reactive oxygen species is significantly different.

Fig. 18 shows the MTT assay of breast tumor cells treated with 40 μM compounds of formula (I) under the existence of 2.5 mM antioxidant NAC. The results show that when cells were only treated with compounds of formula (I), the cell viability is the half of that in the control group. However, when the antioxidant NAC and compounds of formula (I) both existed, the viability of cells was recovered. Thus the effect of compounds of formula (I) on causing cell apoptosis was significantly decreased, and it can be seen that the compounds of formula (I) facilitated apoptosis of breast tumor cells by generating ROS.

### 6. Compounds of formula (I) induce autophagy of breast tumor cells

Cell autophagy is a physiological mechanism where recycle of self-digestion was used as a temporary alternative energy source, but if the cell autophagy was continuous or oversteer-induced, the cell apoptosis may happen later. Some researches pointed out that the cell autophagy and apoptosis interactively regulates each other. In a treatment where cells rebel against induced cell apoptosis, the autophgaic cell death may be used to replace cell apoptosis. Thus it is appropriate to use induced autophgaic cell death as a strategy for treating tumor.

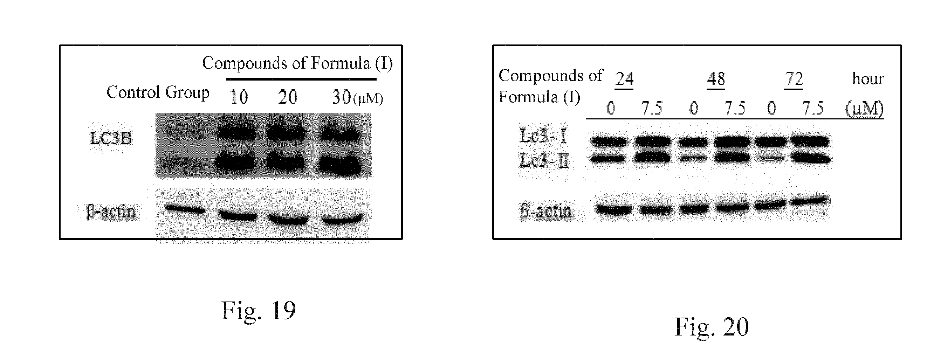

In the following the western blotting was performed to detect the effect of compounds of formula (I) on the autophagy mechanism of breast tumor cells. The results were shown in Figs. 19 and 20.

### Test example 6-1: compounds of formula (I) induce expression of aotophagy-related proteins in breast tumor cells

Fig. 19 illustrates the expression results of proteins LC3B associated with cell autophagy after the breast tumor cells MCF-7 were cultured for 72 h under existence of compounds of formula (I) having different concentrations. The results show that when the concentration of compounds of formula (I) was above 10 μM, the expression amount of proteins LC3B was significantly different with that of the control group. The compounds of formula (I) may be associated with cell autophagy of breast tumor cells MCF-7.

Fig. 20 illustrates the expression results of proteins LC3-II associated with cell autophagy after the breast tumor cells MDA-MB-231 were cultured for 24, 48 or 72 h under existence of compounds of formula (I) having different concentrations. The results show that when the concentration of compounds of formula (I) was 7.5 μM, the expression amount of proteins LC3-II was significantly different with that of the control group. The compounds of formula (I) may be associated with cell autophagy of breast tumor cells MDA-MB-231.

### 7. In animal experiment the compounds of formula (I) inhibit nude mice breast tumor

According to the cell experiments above, it can be seen that compounds of formula (I) have the inhibiting effect on the growth of breast tumor cells. In the following the function of compounds of formula (I) is further vertified through an *in vivo* experiment.

### Test example 7-1: in an in vivo experiment compounds of formula (I) inhibit growth of nude mice breast tumor

The *in vivo* experiment was performed in a nude mice xenograft tumor model. The experimental animals were nude mice of BALB/c-nu species commercially obtained from the National Laboratory Animal Center (NLAC). The nude mice were raise in a germ-free box and the light application time was 7:00-19:00. Filtered air, sterilized water and feed were supplied to and naturally eaten by the nude mice. Female nude mice which matured in 8-10 weeks were used to perform the experiment.

The breast tumor cell (2×10⁶/100 μl) was injected into one side of each back part of 60 nude mice in a hypodermic injection manner. After about 10 days 1-3 mm tumor was grown on each back of the nude mice, the nude mice were randomly divided into 4 groups. Three groups of nude mice were fed with the active ingredient Antrodia camphorata in different doses. The tumor growth was observed and photographed every day. The tumor volume was measured every three days. The volume was calculated according to the equation provided by Osborne et al in 1987.

The experimental animals were divided into the control group (not treated), the solvent group (only physiological saline was injected) and a group injected with compounds of formula (I) (the dose was about 0.75 mg/Kg). The injection was performed everyday, and the animals were fed in normal conditions and photographed for record, so as to compare the tumor growth condition of experimental animals in each group.

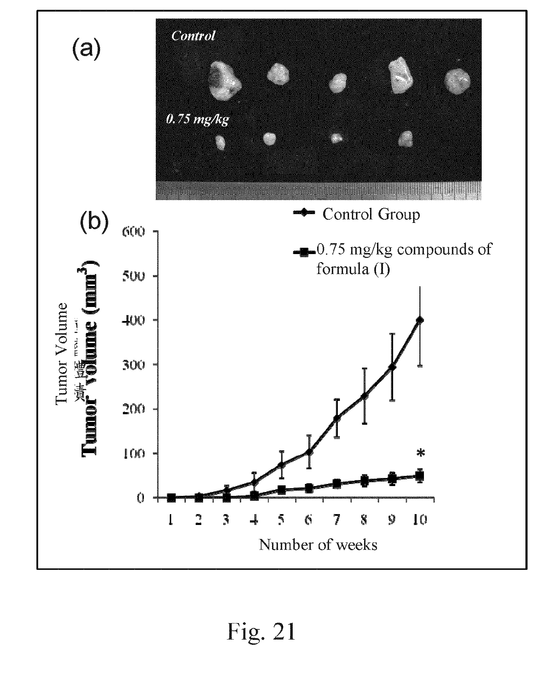

Referring to Fig. 21, section (a) illustrates photographs of tumor volumes of MDA-MB-231 inoculated nude mice in the control group and the group injected with compounds of formula (I); section (b) was a diagram illustrating the relationship between the tumor volume and the growth weeks of MDA-MB-231 inoculated nude mice in the control group and the group injected with compounds of formula (I).

It can be seen from section (a) of Fig. 21 that in the inoculated nude mice injected with compounds of formula (I), the tumor volume was obviously smaller than that of the control group, and a period after the nude mice were injected with compounds of formula (I), the tumor completely disappear.

It can also be obviously seen from section (b) of Fig. 21 that in the inoculated nude mice injected with compounds of formula (I), the tumor volume was obviously smaller than that of the control group. The tumor weight of the control group was 0.455 ± 0.328, and the tumor weight of the injected group was 0.039 ± 0.028, which was obviously different.

### Test example 7-2: histopathological examination and comparison of tumor inoculated nude mice

In the histopathological examination of tumor inoculated nude mice, the inoculated nude mice were examined through a histopathological H&E staining method. The tumor and internal organs were embedded in paraffin and sliced into slides having a thickness of 5 mm by a slicer. Then after deparaffinizing, rehydrating and renaturating, the slide was washed with water twice and stained with Hematoxylin solution for 30-50 s. Then the slide was treated with 0.1% acetic acid-water and washed with water twice. At last the slide was stained with Eosin for 100 s and mounted with Histoclad.

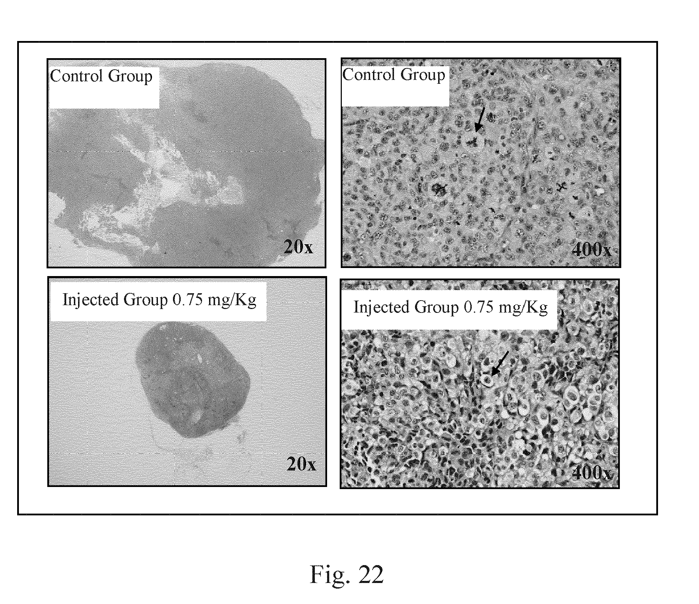

Fig. 22 illustrates photomicrographs taken during the histopathological examination. It can be seen Fig. 22 that in the inoculated nude mice of the control group which were not injected with compounds of formula (I), the tumor volume was large (having a magnification of 20×), and the tumor cells were in a high mitosis state (having a magnification of 400×); and in contrary, in the inoculated nude mice of the group which were injected with compounds of formula (I) at 0.75 mg/Kg everyday, the tumor volume was small (having a magnification of 20×), and the tumor cells were in a low mitosis state (having a magnification of 400×). The results above show that directly injecting compounds of formula (I) in appropriate dose can effectively inhibit tumor cell division.

### Test example 7-3: expression of E-cadherin and Vimentin in cells of tumor inoculated nude mice

The expression amount of signaling-related proteins in the cells of tumor inoculated nude mice was detected by an immunochemistry staining method. In this test the expression of E-cadherin and Vimentin was particularly detected. Immunochemistry staining method and the quantitative method thereof were described as in the test example 2-2, and it is not illustrated any further.

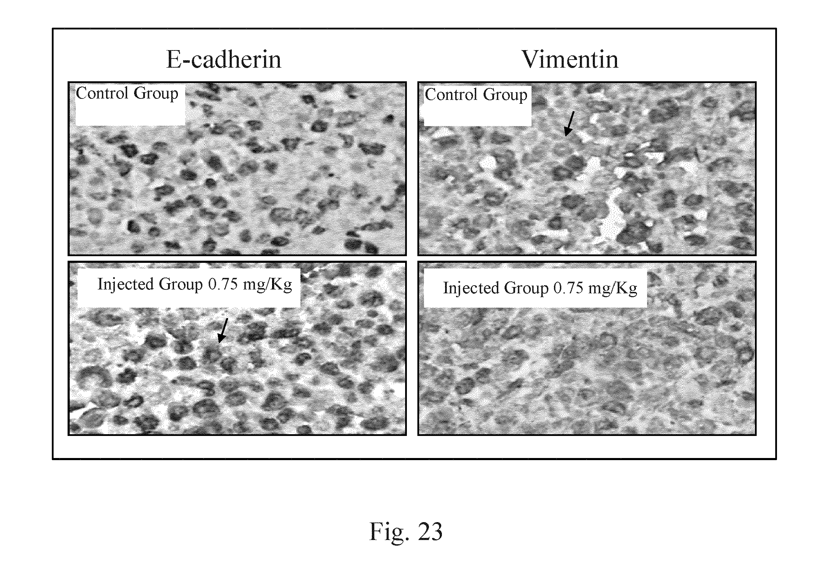

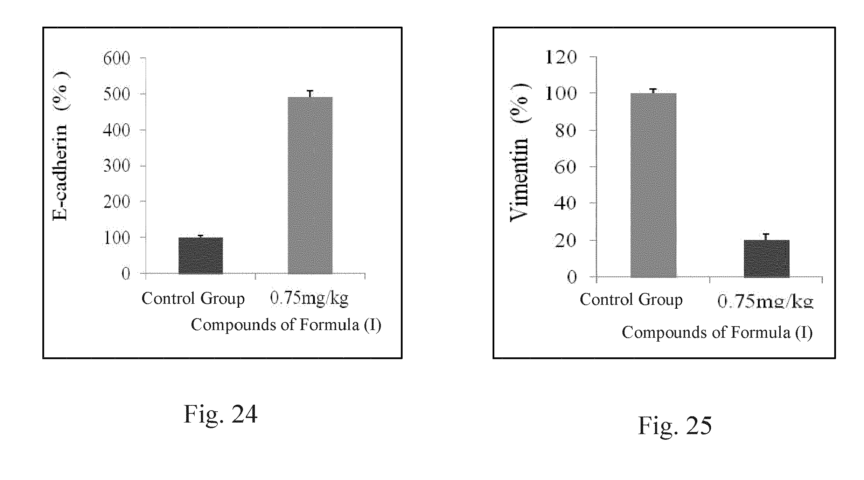

Fig. 23 illustrates photomicrographs of expression results of signaling-related proteins in the cells of tumor inoculated nude mice after the cells were stained through the immunochemistry staining method. Figs. 24 and 25 were respective quantitative results of primary antibody reaction.

The results of Figs. 21, 22 and 23 show that in the inoculated nude mice of the group which were injected with compounds of formula (I) at 0.75 mg/Kg everyday, the expression amount of proteins used for inhibiting tumor proliferation in tumor tissues was high, and the expression amount of proteins used for facilitating tumor proliferation was low. Thus, the compounds of formula (I) had a function of directly inhibiting growth of tumor tissues.

In view of the above, compounds of formula (I) or a mixture including the compounds of formula (I) as the main active ingredient can inhibit tumor cell proliferation. Thus the compounds of formula (I) or a mixture including the compounds of formula (I) as the main active ingredient may be provided as therapeutic drugs in a pharmaceutical composition form.

In the pharmaceutical composition including the compounds of formula (I), the compounds of formula (I) existed in a free form or a pharmaceutically acceptable salt form. Furthermore, the pharmaceutical composition including the compounds of formula (I) may further include one or more pharmaceutically acceptable supporting agents, such as the excipient, solvent, emulsifier, suspending agent, decomposition agent, adhesive agent, stabilizer, antiseptic agent, lubricant agent, absorption delaying agent and lipidosome. The pharmaceutical composition including the compounds of formula (I) was manufactured in an appropriate dosage form depending on actual needs, such as an oral dosage form or an external coating agent form, so as to inhibit tumor cell proliferation.

In view of the above, the compounds of formula (I) and the pharmaceutical composition thereof in the embodiments of the present invention can be applied to inhibit EMT, breast tumor cell metastasis, angiogenesis and expression of Her2/neu and regulate apoptosis/autophagy of breast tumor cells, or can be applied in drugs associated with the functions mentioned above.

Although the present invention has been disclosed with reference to the above embodiments, these embodiments are not intended to limit the present invention. It will be apparent to those of skills in the art that various modifications and variations can be made without departing from the spirit and scope of the present invention. Therefore, the scope of the present invention shall be defined by the appended claims.

## Claims

1. Compounds of following general formula (I) for inhibiting mammalian tumor cell proliferation: 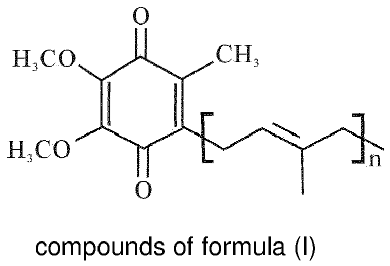 wherein n is an integer from 0 to 9.

2. The Compounds for inhibiting mammalian tumor cell proliferation of claim 1, wherein the cell proliferation of breast tumor, ovarian tumor, skin tumor or liver tumor is inhibited by the compounds of the formula (I).

3. The Compounds for inhibiting mammalian tumor cell proliferation of claim 1, wherein the breast tumor cells are an ERα (estrogen receptor alpha)+ breast tumor cell strain or ERα- breast tumor cell strain.

4. The Compounds for inhibiting mammalian tumor cell proliferation of claim 1, wherein in the compounds of formula (I) n = 0.

5. Pharmaceutical compositions for inhibiting mammalian tumor cell proliferation, comprising the compounds of formula (I) of claim 1, an extract of a processed product or natural product including the compounds of formula (I) as the main active ingredient, the pharmaceutically acceptable salts of the compounds of formula (I), the pharmaceutically acceptable solvate of compounds of formula (I) or any combination thereof.

6. The Pharmaceutical compositions for inhibiting mammalian tumor cell proliferation of claim 5, wherein the processed product is a fermentation liquid of the Antrodia camphorata (Antrodia cinnanonea).

7. The compounds of formula (I) of claim 1, wherein the compounds of formula (I) are applied for inhibiting breast tumor cell proliferation.

8. The compounds of formula (I) of claim 7, wherein inhibiting the breast tumor cell proliferation comprises inhibiting pithelial-mesenchymal transition (EMT).

9. The compounds of formula (I) of claim 7, wherein inhibiting the breast tumor cell proliferation comprises inhibiting metastasis of the breast tumor cell.

10. The compounds of formula (I) of claim 7, wherein inhibiting the breast tumor cell proliferation comprises inhibiting angiogenesis.

11. The compounds of formula (I) of claim 7, wherein inhibiting the breast tumor cell proliferation comprises inhibiting expression of Her2/neu.

12. The compounds of formula (I) of claim 7, wherein inhibiting the breast tumor cell proliferation comprises regulating autophagy of the breast tumor cell.

13. The compounds of formula (I) of claim 1, wherein the compounds of formula (I) are used to manufacture drugs for inhibiting breast tumor cell proliferation.

14. The compounds of formula (I) of claim 13, wherein the manufactured drugs for inhibiting the breast tumor cell proliferation comprise drugs for inhibiting EMT.

15. The compounds of formula (I) of claim 13, wherein the manufactured drugs for inhibiting the breast tumor cell proliferation comprise drugs for inhibiting metastasis of the breast tumor cell.

16. The compounds of formula (I) of claim 13, wherein the manufactured drugs for inhibiting the breast tumor cell proliferation comprise drugs for inhibiting angiogenesis.

17. The compounds of formula (I) of claim 13, wherein the manufactured drugs for inhibiting the breast tumor cell proliferation comprise drugs for inhibiting expression of Her2/neu.

18. The compounds of formula (I) of claim 13, wherein the manufactured drugs for inhibiting the breast tumor cell proliferation comprise drugs for inhibiting autophagy of the breast tumor cell.
